# EUROPEAN PATENT SPECIFICATION

(11) **EP 3 613 395 B1**
(45) Date of publication and mention of the grant of the patent: **02.04.2025**
(21) Application number: 18189970.9
(22) Date of filing: 21.08.2018
(51) Int. Cl.: A61F 13/513, A61F 13/53, A61F 13/531, A61F 13/551, A61F 13/534, A61F 13/537

(54) **ABSORBENT ARTICLES HAVING A CONTRASTING LAYER AND A MASKING LAYER**
ABSORBIERENDE ARTIKEL MIT EINER KONTRASTSCHICHT UND EINER MASKIERUNGSSCHICHT
ARTICLES ABSORBANTS POSSÉDANT UNE COUCHE DE CONTRASTE ET UNE COUCHE DE MASQUAGE

(43) Date of publication of application: 26.02.2020
(73) Proprietor: The Procter & Gamble Company, Cincinnati, OH 45202 (US)
(72) Inventor: GARCIA, Julien Rene, 65824 Schwalbach am Taunus (DE); FRÖHLICH, Ute, 65824 Schwalbach am Taunus (DE); BIANCHI, Ernesto Gabriel, 65824 Schwalbach am Taunus (DE); VAN DER KLUGT, Walter Pieter Hendrik Laurentius, 53881 Euskirchen (DE)
(74) Representative: P&G Patent Germany

(56) References cited:
- WO-A1-2015/031225
- US-A1- 2016 278 986
- US-A1- 2018 116 880

## Description

### FIELD OF THE INVENTION

The invention relates to personal hygiene absorbent articles that are placed in the crotch of a wearer to absorb body exudates, including but not limited to baby taped diapers, baby pant-like diapers (including training pants), feminine pads and adult incontinence products.

### BACKGROUND OF THE INVENTION

Absorbent articles for personal hygiene are designed to absorb and contain body exudates such as urine. These absorbent articles typically comprise a topsheet on the wearer-facing side that is permeable to the fluid and feels soft on the wearer's skin, a backsheet on the garment-facing side for protecting the wearer's clothes, and in-between an absorbent layer for absorbing the fluid. Typical absorbent articles comprise one or more layers between the topsheet and the absorbent core, referred to as acquisition layers, distribution layers or acquisition-distribution layers. These layers have the function to quickly acquire the fluid away from the surface of the article and distribute it to the underlying absorbent core. These acquisitions layers are sometimes colored. Distribution layer comprising loose cross-linked cellulose fibers are used in some commercial products. Acquisition systems comprising a combination of acquisition layers and/or distribution layers are also used in some products, for example as disclosed in US2018/0116880A1 and WO2015/031225A1.

Disposable absorbent articles having color effects and materials thereof are disclosed in US2016/0278986A1. The topsheet therein has a generally planar first surface having a plurality of out-of-plane features and a first color. The layer has a second color, and the plurality of out-of-plane features have a feature color different than the first color and the second color.

The present invention provides a simple and cost efficient way to provide a signal at the wearer-facing side of the article. This signal may be used for example to highlight the presence of channels inside the article.

### SUMMARY OF THE INVENTION

The present invention is for an absorbent article for personal hygiene. The article comprises a topsheet on the wearer-facing side and a backsheet on the garment-facing side. The article comprises an absorbent layer comprising superabsorbent particles and advantageously but optionally cellulose fibers. The absorbent layer is disposed within a core wrap. The absorbent article comprises a masking layer disposed between the topsheet and a contrasting layer. The top layer of the core wrap is the contrasting layer, and the masking layer is a fluid handling layer comprising unbonded or loosely bonded fibers and is free of superabsorbent polymer. The masking layer comprises at least one see-through area so that the contrasting layer is at least partially visible on the wearer-facing side of the article through the see-through area. The contrasting layer has a color that contrasts with the appearance of the masking layer.

The see-through area(s) correspond to channel(s) in the masking layer. In this way, the presence of channels is highlighted at the surface of the article. The contrasting layer may advantageously also have another function than simply providing for the signal at the wearer-facing surface. The contrasting layer is the top layer of a core cover at least partially covering the absorbent material layer. The see-through area and rest of the masking layer have a maximum ΔE* of at least 2.0 as measured on the wearer-facing side of the article, wherein the color difference is measured according to the CIE L*a*b* space method as indicated herein. Such a core cover may be a nonwoven layer comprising fibers with a pigment, or any other contrasting means. The absorbent material may comprise a mixture of cellulose fibers and superabsorbent particles to provide a whiter background for the contrasting layer. This may be advantageous when the absorbent layer does not comprise channels.

The invention may of course comprise further advantageous but not limiting features that are detailed in the following description and the accompanying claims. The absorbent articles of the invention provide in simple manner a contrasting appearance at the wearer-facing side of the article.

### BRIEF DESCRIPTION OF THE DRAWINGS

Fig. 1 is a top view of the wearer-facing side of a taped diaper which has been pulled flat, with some layers partially removed to show the inner layers of the diaper;
Fig. 2 shows an exploded view of the diaper of Fig. 1;
Fig. 3 shows a transversal cross-section of the taped diaper of Fig. 1;
Fig. 4 shows the absorbent core, including the contrasting layer as part of the core cover, superposed with the masking layer comprising the see-through areas and an optional acquisition layer.

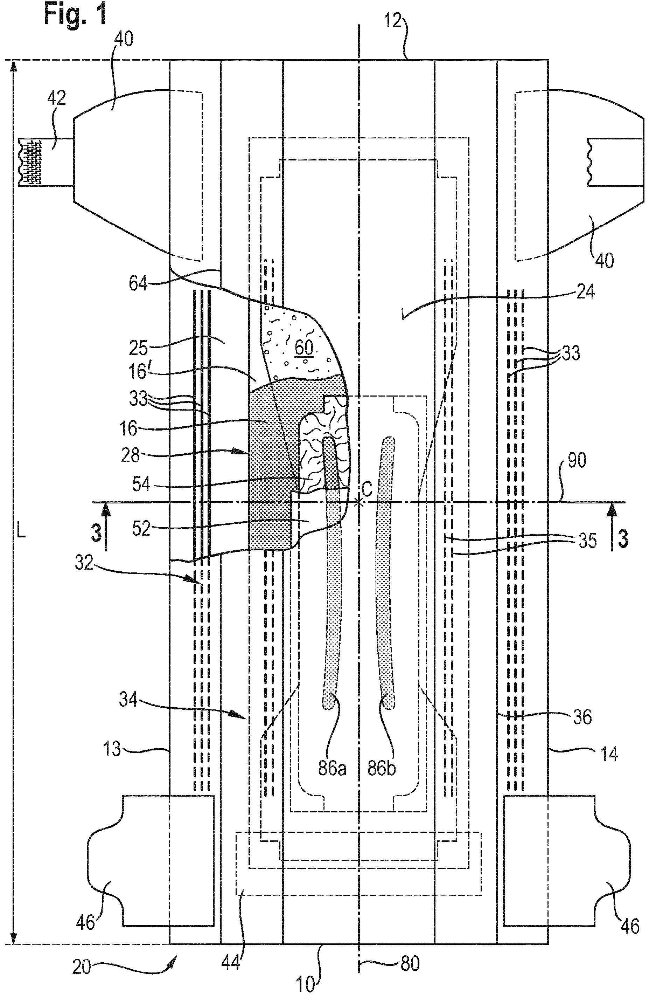

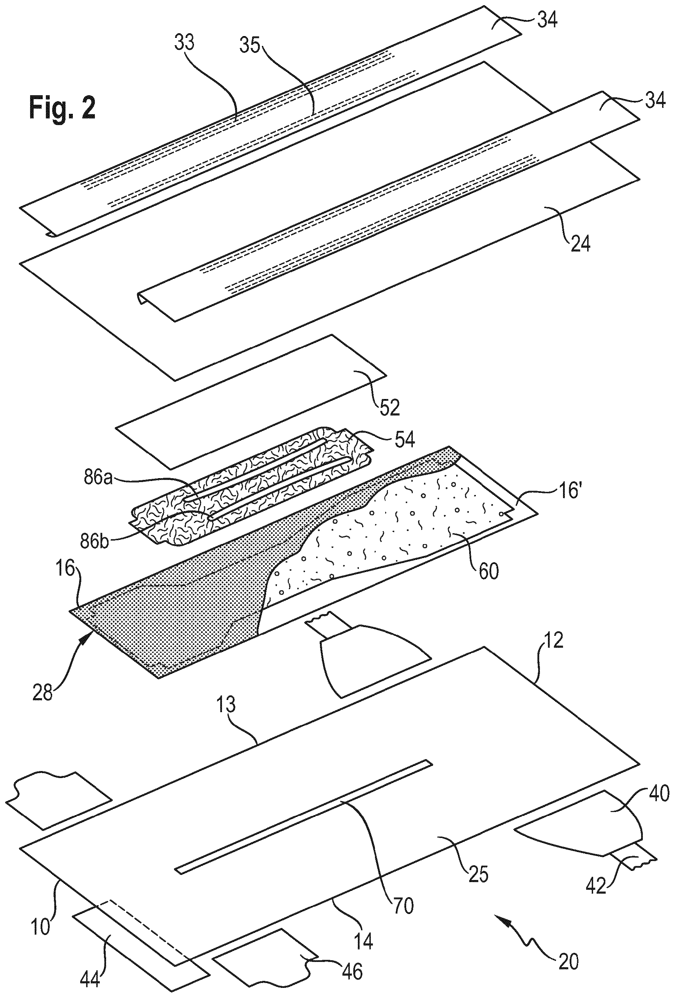

For ease of discussion, the examples of the invention are discussed below with reference to these Figures and the numerals referred therein refer to the article as illustrated in Figs 1-4. The Figures and the numerals are not intended to limit the scope of the claims unless specifically indicated.

### DETAILED DESCRIPTION OF THE INVENTION

### General description of an absorbent article 20

An exemplary absorbent article according to the invention in the form of a baby taped diaper 20 is represented in Figs. 1-3. This diaper 20 is shown for illustration purpose only, as the invention may be used for making a wide variety of diapers or other absorbent articles such as pant-like diapers, training pants, adult incontinence pants or feminine sanitary pads. In the following description the term diaper and absorbent article are used interchangeably.

As illustrated in Fig. 1, the absorbent article 20 comprises a front edge 10, a back edge 12, and two longitudinally-extending side (lateral) edges 13, 14. The front edge 10 is the edge of the article which is intended to be placed towards the front of the user when worn, and the back edge 12 is the opposite edge. The absorbent article is notionally divided by a longitudinal axis 80 extending along a longitudinal direction from the middle of the front edge to the middle of the back edge of the article and dividing the article in two substantially symmetrical halves relative to this axis, when viewing the article from the wearer-facing side in a flat-out configuration, as exemplarily shown in Fig. 1. If some parts of the article are under tension due to elasticized components, the article may be typically flattened using clamps along the periphery of the article and/or attached to a sticky surface, so that the article can be pulled taut so as to be substantially flat. Closed articles such as pant-like baby diapers, training pants for small children, or adult incontinent pants may be cut open along the side seams to apply them on a flat surface, as is known in the art. Unless otherwise indicated, dimensions and areas disclosed herein apply to the article in this flat-out configuration.

The article has further a length L as measured along the longitudinal axis 80 from the back edge 12 to the front edge 10. The absorbent article can also be notionally divided by a transversal axis 90 at half the length L. The transversal axis 90 is perpendicular to the longitudinal axis 80 and placed at half the length of the article. The intersection of the longitudinal axis 80 and the transversal axis 90 is defined herein as the centerpoint C of the article. The article can be further notionally divided in three regions having equal length of a third of L along the longitudinal axis: a front region extending from the front edge 10 towards the crotch region for a third of L, a crotch region in the middle third of the diaper, and a back region extending from the crotch region to the back edge 12 of the article for the remaining third of L. All three regions are of equal length measured on the longitudinal axis, when the article is in such a flat state. The front region, crotch region, back region and longitudinal and transversal axis are defined herein notionally, that is they are typically not materialized in the real diapers, but are useful to describe the positions of various components of the invention relative to each other and the diaper.

The absorbent article 20 comprises a liquid-permeable topsheet 24, a liquid-impermeable backsheet 25. The topsheet 24 typically forms most of the wearer-contacting surface of the article and is the first layer that the body exudates contact. The topsheet is preferably compliant, soft-feeling, and non-irritating to the wearer's skin. Further, at least a portion of the topsheet is liquid permeable, permitting liquids to readily penetrate through its thickness. Any known topsheet may be used in the present invention. A suitable topsheet may be manufactured from a wide range of materials. Most topsheets are nonwoven materials or apertured formed films, but other materials are possible such as porous foams, reticulated foams, woven materials. Typical diaper topsheets have a basis weight of from about 10 gsm to about 28 gsm, in particular from about 12 gsm to about 18 gsm but higher basis weights are possible if it is desired to provide a very soft feeling wearer-contacting surface for example.

The backsheet may be any backsheet known in the art for absorbent articles. The backsheet may be positioned directly adjacent the garment-facing surface of the absorbent core. The backsheet prevents, or at least inhibits, the exudates absorbed and contained therein from soiling articles such as bedsheets and undergarments. The backsheet is typically impermeable, or at least substantially impermeable, to liquids (e.g., urine). The backsheet typically comprises a thin plastic film, such as a thermoplastic film having a thickness of about 0.012 mm to about 0.051 mm. The basis weight of those films is usually as low as possible to save material costs, typically from 10 gsm to 30 gsm, in particular below 20 gsm. A covering low basis weight nonwoven may be attached to the external surface of the film to provide for a softer touch. The backsheet may be breathable, as is known in the art.

The absorbent article comprises an absorbent layer 60 between the topsheet and backsheet. The absorbent layer comprises an absorbent material. The absorbent material comprises a superabsorbent polymer, which may in particular be a mixture of superabsorbent particles with cellulose fibers. The absorbent layer is typically disposed within a core wrap, together forming an absorbent core. The absorbent core comprises the absorbent material layer 60 and the core wrap having a top side 16 and bottom side 16'. The periphery of the absorbent layer 60 also defines a deposition area.

### Masking layer 54 and see-through areas 86

Referring now with a first execution as exemplified in the example of Figs. 1-4, the absorbent article comprises a masking layer 54 between the topsheet 24 and the absorbent material layer 60. The masking layer 54 comprises at least one see-through area 86, for example two see-through areas 86a, 86b, as shown in the Figures. The masking layer material has a certain opacity so that it can at least partially hide the contrasting layer 16 disposed underneath, except of course in the see-through areas 86. See-through areas are discrete areas within the masking layer that are transparent or more translucent than the rest of the masking layer, so that the contrasting layer is at least partially visible on the wearer-facing side of the article through the see-through area. The Figures show two such see-through areas 86a, 86b disposed symmetrically relative to the longitudinal axis 80, but this is of course non-limiting of the numbers, shapes or placement of the see-through areas. In the following description, the plural "see-through areas" will be used to mean "one, two or more see-through areas", unless it is clear that a more precise number is meant.

The masking layer 54 material is advantageously relatively opaque outside the see-through areas, to increase the contrast between the see-through areas and the rest of the masking layer when considered from the wearer-facing surface of the article. The masking layer may also be advantageously relatively thick outside the see-through areas, to provide for a three-dimensional tactile appearance at the wearer-facing surface of the absorbent article.

For these reasons, the masking layer may advantageously have a relatively high basis weight, for example an average basis weight of at least 50 g/m², in particular from 50 g/m² to 400 g/m², and advantageously of at least 100 g/m². The average basis weight is calculated by dividing the weight amount of the fibers by the area of the distribution layer where the fibers are present (including the see-through areas). The masking layer may have a relatively low density (high bulk) to provide for loft and opacity. The density of the layer may vary depending on the compression of the article, but may typically range from 0.03 g/cm³ to 0.25 g/cm³, in particular from 0.05 g/cm³ to 0.15 g/cm³, measured at 0.30 psi (2.07kPa) on an area of the masking layer outside the see-through area. The density of the masking layer may be measured at the centerpoint C of the article for this purpose, unless this is area is in a see-through area then at the closest from the centerpoint C on the longitudinal centerline.

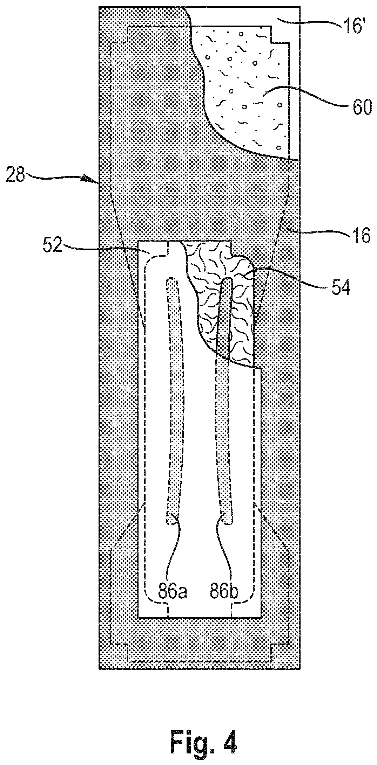

The masking layer 54 has advantageously at least a second function to reduce the use of raw material. The masking layer may thus advantageously be made of a distribution or acquisition layer material to provide fluid-handling properties in addition to the masking effect. The masking layer can thus advantageously spread an insulting fluid over a larger surface within the article so that the absorbent capacity of the core can be more efficiently used. Such a masking layer may be smaller in surface than the absorbent layer and typically does not extend beyond the edges of the absorbent layer, as exemplarily illustrated in Fig. 4.

The masking layer does not require to be a nonwoven layer, in particular the masking layer comprises or consists of loose fibers with no or weak intra-fiber bonds. A typical example of such material comprises or consists of cross-linked cellulose fibers. The masking layer may for example comprise at least 50% by weight of cross-linked cellulose fibers. The cross-linked cellulosic fibers may be crimped, twisted, or curled, or a combination thereof including crimped, twisted, and curled.

Examples of such chemically cross-linked cellulosic fibers that have been used to make distribution layer are disclosed in US5,549,791, US5,137,537, WO95/34329 or US2007/118087. This type of material has been used in the past in disposable diapers as part of an acquisition-distribution system, for example in US2008/0312622 A1 (Hundorf). The distribution layer comprising cross-linked cellulose fibers may comprise other fibers, but this layer may comprise at least 50%, or 60%, or 70%, or 80%, or 90% or even up to 100%, by weight of the layer, of cross-linked cellulose fibers (including the cross-linking agents). The cross-linked cellulosic fibers provide higher resilience and therefore higher resistance against the compression in the product packaging or in use conditions, e.g. under baby weight. Cellulose fibers also have an opaque white appearance which increase the contrast with the see-through areas that may be substantially free of these fibers. While the distribution material may be comprised of cellulose fibers, in particular cross-linked cellulose fibers, other materials are of course possible.

Such a fibrous material layer comprising relatively loose fibers and having see-through areas may be manufactured by air-laying the fibers on a drum comprising several molds each having the required depth profile for the desired fibrous material configuration. The formed masking layer can then be directly un-molded onto another component of the article such as a nonwoven carrier layer or an acquisition layer and then integrated with the rest of the article in a known manner. When a nonwoven acquisition layer 52 is present in the article, the masking layer 54 may be for example deposited on this acquisition layer, the two layers being further joined to absorbent core and the rest of the article, as is known in the art.

The see-through areas are channel areas substantially free of masking layer material. By "substantially free", it is meant that some material may be present, such as unavoidable contamination during the high speed making process, but that this inadvertently present material does not provide a noticeable function to the area. Of course these channel areas may be completely free of masking layer material. This has the advantage that the topsheet can recess within the see-through areas and provide a three-dimensional appearance at the wearer-facing side of the article. Absorbent articles having a three-dimensional surface on their wearer-facing surface have been proposed commercially (e.g. as disclosed WO2015/031225A1, Roe et al.). The three-dimensional surface at the surface of the article can help keeping the skin of the user dry, among other benefits such as better fluid handling or distribution.

The see-through areas 86 may have any desired shapes and positions within the masking layer 54. There may be one, two, three, four or more discrete see-through areas. The see-through areas may be straight, including parallel to the longitudinal axis and/or angled relative to the longitudinal axis, or the see-through areas may be curved, or a combination of both straight and curved, etc... There may be for example only one discrete see-through area comprising several branches, for example having a U or V shape comprising two branches which meet at one extremity, or having a O shape with both extremities connected, or a X shape, or Y shape etc... The see-through areas may have a minimum width of for example at least 2 mm, or at least 4 mm and a maximum width of for example up to 20 mm, or up to 12 mm. A typical width may be from 6 to 8 mm. If the see-through area's width varies significantly along their length, the value indicated may thus be present in at least one see-through area section having a length of at least 20 mm. The see-through areas may have any length, for example from 50 mm to 400 mm, as measured projected along the longitudinal axis 80. It is also possible to have a see-through area formed by a plurality of shorter segments separated by buffer zones. The see-through areas may be typically longitudinally oriented, that is at least twice longer in the longitudinal direction than in the transversal direction. The see-through areas 86 may also advantageously not extend to the edges of the masking layer 54 but remain fully encompassed with the masking layer. The see-through areas are typically also fluid-directing channels. They may be thus at least present or extend in the crotch area of the article, for example in the crotch area and front area, or the crotch area and back area, or all three areas.

### Contrasting layer 16

The absorbent article comprises a contrasting layer 16 disposed underneath the masking layer 54 and the see-through areas 86. The contrasting layer has a visible appearance that contrasts with the rest of the article, in particular that contrasts with the appearance of the masking layer. The contrasting layer may be treated or manufactured to have a color (e.g. any shades of green, blue, yellow, brown, grey, red etc..) while the masking layer is generally untreated so that it retains a generally white appearance (as is common for cellulosic fibers or non-colored plastics), or wherein the masking layer has a different color. An example of contrasting layer is a nonwoven layer made of synthetic fibers which have been colored by the addition of a pigment during its manufacture. The contrasting layer is at least partially visible on the wearer-facing surface of the article through the see-through areas and thus can provide a visual signal highlighting the presence and position of the see-through areas, which may be three-dimensional channels in the masking layer, as indicated above. The contrasting layer may also have a different appearance caused by having a different texture, instead or in addition to a different color, for example a three-dimensional pattern may be embossed or molded on the contrasting layer.

Color may be imparted to a contrasting layer by way of impregnation of a colorant into the substrate material. Colorants such as dyes, pigments, or combinations may be impregnated in the formation of substrates such as polymerics, resins, or nonwovens. For example, the colorant may be added to molten batch of polymer during film, fiber, or filament formation. EP2,886,093A1 (Kreuzer et al.) discloses various ways to provide color or another contrasting means to a nonwoven layer, which are also applicable herein to make the contrasting layer, and in particular include adding a pigment in a synthetic uncolored material which is then manufactured in a nonwoven (compounding or master-batching). Other applicable contrasting methods are also included such as printing or coating a contrasting layer on whole of the contrasting layer or on selected areas corresponding to the see-through areas.

Typical topsheets (and optionally present acquisition layers) have a low basis weight and are translucent so that the contrasting layer is visible through these layers. The contrasting layer may have the sole function of providing a visual signal at the surface of the article through the see-through areas, but it is of course preferable that it also has another function for material savings and cost reasons. The contrasting layer may in particular be part of the absorbent core 28, in particular it may be part of the core wrap which is typically provided to sandwich the absorbent material. The core wrap is typically a substrate used for receiving and stabilizing the absorbent material when the core is made and comprises a top side 16 and a bottom side 16'.

Various core wrap constructions are possible. The core wrap may in particular comprise as represented in the Figures two separate substrates 16, 16' forming the top side and the bottom side of the core wrap respectively. The top layer may be called core cover or core wrap cover. Two substrates forming the core wrap may be longitudinally attached in a C-wrap or alternatively in a sandwich configuration (as represented) with the two substrates in a face to face relationship along the two longitudinal seals. A front end seal and a back end seal may be optionally present. However, this core wrap construction is not limiting of the invention, as any conventional core wrap construction may also be used, for example a single substrate on a portion of which the absorbent material is deposited and then the rest of the substrate folded over the deposited absorbent material to form the other side of the core. This single substrate construction can then be sealed longitudinally with a single longitudinal edge seal.

The contrasting layer is the top layer of the core wrap, which is a cover material suitable for receiving and containing the absorbent material. The basis weight does not need to be high, and may for example range from 5 gsm to 50 gsm, in particular from 5 gsm or from 8 gsm or from 10 gsm up to 40 gsm or 30 gsm. Typical substrate materials may be paper, tissues, films, wovens or nonwovens, or laminate of any of these. The core wrap may in particular be formed by a nonwoven web, such as a carded nonwoven, spunbond nonwoven ("S") or meltblown nonwoven ("M"), and laminates of any of these. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US7,744,576, US2011/0268932A1, US2011/0319848A1 and US2011/0250413A1. Nonwoven materials are typically made of synthetic fibers, such as PE, PET and in particular PP fibers.

### Optional acquisition layer 52

An optional acquisition layer 52 may be present in the absorbent article, for example as shown in the Figure directly under topsheet to quickly acquire any fluid away from the topsheet. The acquisition layer may for example also serve as support substrate for the masking layer underneath when this layer is not a nonwoven, in particular when it is made of cellulose fibers as described above. The optional acquisition layer may have the same length as the masking layer, especially if these are made continuously, with the continuous layers combined and individualized simultaneously. The optional acquisition layer may be for example a nonwoven layer having a basis weight of from 10 gsm to 100 gsm, typically from 10 gsm to 60 gsm.

The acquisition layer material is typically a nonwoven material. As used herein, the terms "nonwoven material", "nonwoven layer", "nonwoven web" or more simply "nonwoven" are defined as a sheet of fibres, continuous filaments, or chopped yarns of any nature or origin, that have been formed into a web by any means, and bonded together by any means, with the exception of weaving or knitting (ISO 9092 definition). Felts obtained by wet milling are not nonwovens. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m² or gsm).

Nonwovens have the advantage that they can be manufactured outside the converting line and stored and used as a roll of material. A typical nonwoven layer that may be used in the present invention as acquisition layer is a bonded carded web, in particular a through-air bonded carded web ("TABCW"). "Bonded carded web" refers to webs that are made from staple fibers that are sent through a combing or carding unit, which breaks apart and aligns the staple fibers in the machine direction to form a generally machine direction-oriented fibrous nonwoven web. This web is then drawn through a heated drum, creating bonds throughout the fabric without applying specific pressure (through air bonding process). A TABCW material provides a low density, lofty through-air bonded carded web. The web may for example have a basis weight at from about 15 gsm to about 120 gsm (gram per square meter), in particular from about 30 gsm to about 80 gsm. A TABCW material can for example comprise about 3 to about 10 denier staple fibers. Examples of such TABCW are disclosed in WO2000/71067 (KIM DOO-HONG et al.). TABCW are available directly from all usual suppliers of nonwoven webs for use in absorbent articles, for example Fitesa Ltd or Fiberweb Technical Nonwovens.

The acquisition material layer may also be a latex bonded nonwoven. Examples of such acquisition layers are disclosed in US7,786,341 (Schneider et al.). Carded, resin-bonded nonwovens may be used, in particular where the fibers used are solid round or round and hollow PET staple fibers (50/50 or 40/60 mix of 6 denier and 9 denier fibers). An exemplary binder is a butadiene/styrene latex. Further useful nonwovens are described in US6,645,569 (Cramer et al.), US6,863,933 (Cramer et al.), US7,112,621 (Rohrbaugh et al.), US2003/148684 (Cramer et al.) and US2005/008839 (Cramer et al.). The acquisition layer may be stabilized by a latex binder, for example a styrene-butadiene latex binder (SB latex). Processes for obtaining such latex are known, for example, from EP 149880 (Kwok) and US 2003/0105190 (Diehl et al.). The binder may typically be present in the acquisition layer in amount ranging from about 12% to about 50%, for example about 30%, by total weight of the acquisition layer. SB latex is available under the trade name GENFLO^{™} 3160 (OMNOVA Solutions Inc.; Akron, Ohio).

### Absorbent core 28 and absorbent layer 60

The layer of absorbent material is typically included in a discrete absorbent core 28, which is the component of the article that has the maximum fluid retention capacity. The absorbent core 28 comprises the absorbent layer 60, which is contained in a core wrap. Various core constructions are known and may be used herein. As used herein, the term "absorbent core" does not include the topsheet, the backsheet or a distribution/acquisition layer. The absorbent layer comprises all or at least the majority of superabsorbent polymer (SAP) in the article. The absorbent core thus typically consists essentially of, or consists of, the core wrap, the absorbent material and optionally construction adhesives. The absorbent material advantageously comprises or consists of a blend of SAP particles and cellulose fibers, as the cellulose fibers can provide a white, opaque background under the contrasting layer, but the invention is also applicable to other absorbent material for example consisting to 100% of SAP particles. The terms "absorbent core" and "core" are herein used interchangeably.

The absorbent layer 60 comprises superabsorbent polymers (SAP), as is known in the art. Suitable SAP may be any water-insoluble, water-swellable polymers capable of absorbing large quantities of fluids, as is known in the art. The term "superabsorbent polymer" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of more than 20 g/g, or more than 24 g/g, or of from 20 to 50 g/g, or from 20 to 40 g/g, or 24 to 30 g/g.

The SAP is typically formulated in the form of particles, which may be distributed in a matrix of cellulose fibers. The SAP typically represents from 40% to 80%, in particular from 50% to 70%, of the weight of the absorbent material, the rest being cellulose and/or synthetic fibers. More recently, so called pulp-less or airfelt-free absorbent cores have been put on the market, wherein the absorbent material does not comprise cellulose fibers. In these airfelt-free cores, the SAP particles have been enclosed in pockets, see for example US5,433,715 (Tanzer et al.), WO2012/052172 (Van Malderen), or have been immobilized by a fibrous network of adhesive fibers (e.g. US2008/312617, Hundorf et al.).

The absorbent material layer 60 defines an absorbent material deposition area delimited by the periphery of the absorbent layer formed by the absorbent material, as seen from above within the plane of the core. The deposition area may be advantageously shaped so that the longitudinal edges of the cores have a tapered section in the crotch region relative to the front region and/or back region, as is illustrated in the Figures, but the absorbent layer may also be generally rectangular. An inner core glue between the top side and bottom side of the core wrap is optional but advantageous to improve the adhesion between the inner surfaces of the core wrap and the absorbent material.

Absorbent cores comprising one, two or more macroscopic channels have been proposed (for example in WO2012/170778A1, Rosati et al., or WO 2014/200794 Al, Bianchi et al). These channels are typically elongated areas within the absorbent material layer deposition area that are free substantially free or free of absorbent material. The channels and encompassed within the absorbent material layer. However, it can be advantageous in the present invention that the absorbent material layer does not comprise such channels, or at least does not comprise such channels in areas which are superposed with the see-trough area. This is benefits from the inherent opacity of the absorbent material layer 60 to provide for a more homogenous background for the contrasting layer disposed above.

Other optional components of the absorbent article that are typically present in commercial products are briefly discussed below.

Absorbent cores comprising one, two or more macroscopic absorbent material-free channels have been proposed (for example in WO2012/170778A1, Rosati et al., or WO 2014/200794 Al, Bianchi et al). These channels are typically elongated areas within the absorbent material layer deposition area that are (substantially) free of absorbent material. The channels and encompassed within the absorbent material layer. However, it can be advantageous in the present invention that the absorbent material layer does not comprise such channels, or at least does not comprise such channels in areas which are superposed with the see-trough area when the masking layer 54 is disposed above the absorbent layer 60 (as shown in Figs.1-4). This benefits from the inherent opacity of the absorbent material layer 60 to provide for a more homogenous background for the contrasting layer disposed above.

The following will describe other typical elements of a diaper, which may be optionally also present in the articles of the invention.

### Fastening system 42, 44

The absorbent article may include a fastening system, especially when the article is a taped diaper as exemplified in Fig. 1. The fastening system can be used to provide lateral tensions about the circumference of the absorbent article to hold the absorbent article on the wearer. Such a fastening system is not necessary for pant articles such as training pants and adult incontinence pants since the waist region of these articles is already bonded and elasticized. The fastening system usually comprises a fastener 42 such as tape tabs, hook and loop fastening components, interlocking fasteners such as tabs & slots, buckles, buttons, snaps, and/or hermaphroditic fastening components, although any other known fastening means are generally acceptable. A landing zone 44 is normally provided on the front waist region of the article for the fastener 42 to be releasably attached. Some exemplary surface fastening systems are disclosed in US3,848,594, US4,662,875, US4,846,815, US4,894,060, US4,946,527, US5,151,092 and US5,221,274 (Buell).

### Front and back ears 46, 40

The absorbent article may comprise front ears 46 and back ears 40 as is known in the art in taped diapers. Absorbent articles in pant chassis are already sealed along the waist edges and typically do not require front ears and back ears. The ears can be integral part of the chassis, for example formed from the topsheet and/or backsheet as side panel. Alternatively, as represented in Fig. 1, they may be separate elements attached by gluing and/or heat embossing. The back ears 40 are optionally stretchable to facilitate the attachment of the tabs 42 on the landing zone 44 and maintain the taped diapers in place around the wearer's waist. The front ears 46 may also be optionally elastic or extensible to provide a more comfortable and contouring fit.

### Barrier leg cuffs 34 and gasketing cuffs 32

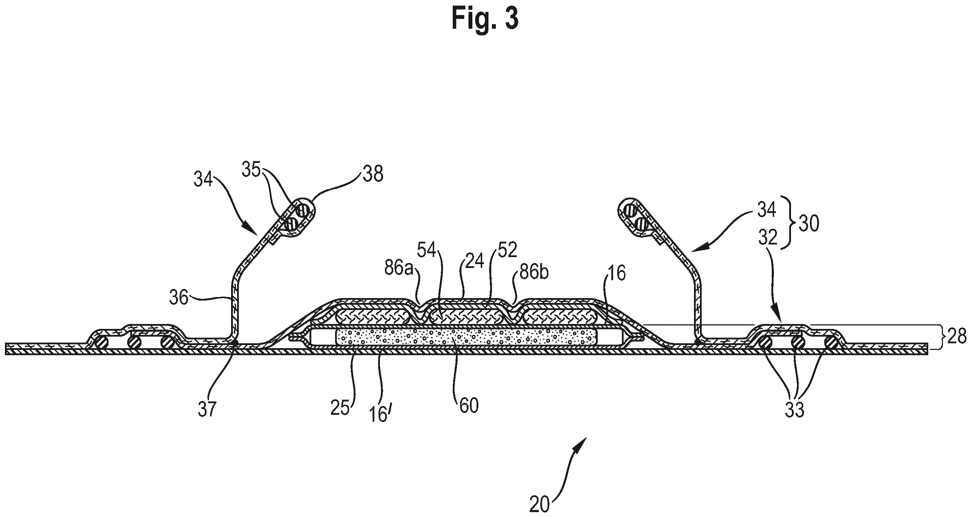

Absorbent articles such as taped diapers, training pants or adult incontinence pants may typically further comprise cuff components 30 that improve the fit of the article around the legs of the wearer. Such cuffs typically comprise barrier leg cuffs 34 and gasketing cuffs 32. The cuffs 30 may comprise a piece of material, typically a nonwoven, which is one side partially bonded to the article and on the other side can be partially raised away from the topsheet and thus stand up from the plane defined by the topsheet as shown for example in Fig. 3. Both parts of the cuffs may be advantageously elasticized. The raised part of the cuff components is referred to herein as barrier leg cuffs 34 and can provide improved containment of liquids and other body exudates approximately at the junction of the torso and legs of the wearer. The barrier leg cuffs 34 extend at least partially between the front edge and the back edge of the absorbent article on opposite sides of the longitudinal axis and are at least present adjacent to the center point C of the article.

The barrier leg cuffs 34 may be delimited by a proximal edge 37 joined to the rest of the article, typically the topsheet, and a free terminal edge 38 intended to contact and form a seal with the wearer's skin. The barrier leg cuffs 34 may be joined at the proximal edge 37 with the chassis of the article by a bond which may be made for example by adhesive bonding, fusion bonding or combination of known bonding means, for example as disclosed in WO2014/168810A1 (Bianchi et al.). The bond at the proximal edge 37 may be continuous or intermittent.

The barrier leg cuffs 34 can be integral with (i.e. formed from) the topsheet or the backsheet, or more typically be formed from a separate material joined to the rest of the article. Typically, the material of the barrier leg cuffs may extend through the whole length of the article but is "tack bonded" to the topsheet towards the front edge and back edge of the article so that in these sections the barrier leg cuff material remains flush with the topsheet. Each barrier leg cuff 34 may comprise one, two or more elastic strings 35 close to its free terminal edge 38 to provide a better seal.

In addition to the barrier leg cuffs 34, the article may comprise gasketing cuffs 32, which are formed in the same plane as the chassis of the absorbent article, in particular may be at least partially enclosed between the topsheet and the backsheet, and typically placed further laterally outwardly relative to the barrier leg cuffs 34. The gasketing cuffs 32 can provide a better seal around the thighs of the wearer. Usually each gasketing leg cuff 32 will comprise one or more elastic string or elastic element 33 comprised in the chassis of the diaper, for example disposed between the topsheet and backsheet in the area of the leg openings. Typically, the barrier leg cuffs 34 are disposed more internally than the gasketing cuffs 32. The barrier leg cuffs are thus also referred to as inner cuffs and the gasketing cuffs as outer cuffs.

### Other components

A lotion (not represented) may be present, typically in longitudinally-extending slots, directly on the topsheet. Some typical diaper components are represented in the Figures, such as elasticized gasketing cuffs 32 (also called outer cuffs) comprising elastics strands 33, upstanding barrier leg cuffs 34 (inner cuffs) comprising elastic strands 35 as is known in the art. Typically each cuff may typically comprise from 1 to 4 elastic strands. In taped diapers, a pair of fastening tabs 42 and a landing zone 44 are typically provided. The absorbent article may also comprise other typical components, which are not represented in the Figures, such as a back elastic waist band, a front elastic waist band, transverse barrier cuffs, a wetness indicator 70 between the backsheet and the absorbent core that changes color when contacted with urine, etc...

### Pant-like articles

As indicated previously, the invention may be also used in absorbent articles presented in the form of a pant or underwear (herein "pant"). In these articles, the waist and the leg openings are pre-formed during manufacture so that the article can be put on like underwear. These pant articles typically have a front waist panel and a back waist panel which are sealed together via side seams. The side seams can be broken to remove and discard the article and are typically not re-fastenable. The front and back waist panels are typically elasticized. Pants can be used as taped diapers on babies and younger children for day wear and for overnight dryness, or as training pant for older children at the toilet training stage, and also as adult incontinence protection.

Pant-like articles typically comprise a front waist panel and a back waist panel joined together via side seams to form the waist opening and at least part of the leg openings. The waist panels are typically elasticized, either using a material which is inherently elastic to make them (such as a laminate comprising an elastomeric layer between two nonwoven layers) or by sandwiching a plurality of elastic strands between two nonwovens along the width of the panels, as is known in the art. Pants also typically comprise a chassis comprising the remaining components of the article, in particular the topsheet, the backsheet, the absorbent core and barrier cuffs including upstanding barrier leg cuffs and attached on one side to the front waist panel and on the other side of the back waist panel. These components may be generally constructed as in previously disclosed for the taped diaper.

### Packages

A plurality of articles according to the invention may be packaged in a package for transport and sale. At least 50% of the articles, and preferably all the articles, in the package may be according to the invention. The articles may be folded and packaged as is known in the art. The package may be for example a plastic bag or a cardboard box. Diapers may typically be bi-folded along the transversal axis and the ears folded inwardly before being packaged. The absorbent articles may be packed under compression so as to reduce the size of the packages, while still providing an adequate number of absorbent articles per package. By packaging the absorbent articles under compression, caregivers can easily handle and store the packages, while also providing distribution and inventory savings to manufacturers owing to the size of the packages.

### Relations between the layers and process for making

Typically, adjacent layers will be joined together using conventional bonding method such as adhesive coating via slot coating or spraying on the whole or part of the surface of the layer, or thermo-bonding, or pressure bonding or combinations thereof. Most of the bonding between components is for clarity and readability not represented in the Figure. Bonding between the layers of the article should be considered to be present unless specifically excluded. Adhesives may be typically used to improve the adhesion of the different layers, for example between the backsheet and the core wrap. The adhesives used may be any standard hotmelt glue as known in the art. The individual components may be converted into an absorbent article according to any of the processes known in the art.

The topsheet may be advantageously is directly or indirectly bonded to an underlying layer through the see-through areas, especially when these are channels free or substantially free of absorbent or distribution material. Referring to Fig. 3 for example, the topsheet 24 may be glued or otherwise bonded to an underlying layer 54 which is itself bonded through the see-through areas 86 to the underlying layer, in this example the masking layer / core wrap 16.

### Example

A taped baby diaper as generally constructed as shown in Figs. 1-3 and comprising an absorbent core and an acquisition layer/masking layer composite as shown in Fig. 4 was made on a diaper converting line. The masking layer was made of air-laid cross-linked cellulose fibers. In this example, the average basis weight of the masking layer was ca. 265 gsm. More generally, the basis weight of the masking layer can typically range from 50 gsm to 400 gsm. The masking layer had two see-through areas 86a,b which are areas free of masking material. These channels were longitudinally extending and slightly inwardly curved. The curved channels were approximately 15 cm long and 6 mm wide.

The contrasting layer in this example was a SMS (spunbond-meltblown-spunbond laminate) PP nonwoven with a 10 gsm basis weight. The contrasting layer had a teal color provided by teal pigments used in the plastic matrix (supplier: UNION, Product Code DXX03PPHA). More generally, any colored nonwovens for example having a basis weight ranging from 5 gsm to 30 gsm could be used. The absorbent core was a shaped core (non-rectangular) without channels and comprising a mix of cellulose fibers and superabsorbent polymers (about 67% to SAP, more generally the %SAP may for example range from 40% to 90%, for example from 50% to 80%). The specific values indicated above are average, or target value, as it is of course known that in modern productions of diapers made at very high speed some variations may happen between individual diapers made on the line.

The topsheet was a nonwoven having a basis weight of ca. 15 gsm. An acquisition layer 52 in the form a latex bonded nonwoven having a basis weight of ca. 43 gsm was also provided, between the masking layer 54, as generally illustrated in the Figures. Both the topsheet and the masking layer have a generally white translucent appearance. The masking layer had a generally white appearance, except in the see-through areas which were transparent.

Color measurements (according to CIE L*ab*, as described below) were made on the wearer-facing surface of the article on a region of the topsheet corresponding to the see-through areas and a region of the topsheet corresponding to the masking layer outside the see-through areas to determine a quantitative value for color difference, referred to in the art as ΔE*. In the present example, the areas corresponding to the different layers of interest appeared generally homogenous so that the exact region for each measurement was not critical. If, on the other hand, the see-through areas and/or the masking layer were substantially not homogenous, the measurements can be made on a region of each area having the most apparent contrast to obtain a maximum ΔE* value.

The average value (n=10) measured at the surface of the topsheet on a region corresponding to the masking layer was L* = 74.36, a* = -4.76 and b* = 2.07, while the average value (n=10) measured on the topsheet for a region corresponding to the see-through area was L* = 68.67, a* = -7.78 and b* = 0.51. The ΔE* value between the two regions can be calculated to be 6.62. Typically, a ΔE* difference of 2 is considered to indicate two substantially different colors.

The see-through area and the rest of the masking layer have a ΔE* of at least 2.0, or at least 3.0, or at least 4.0, as measured on the wearer-facing side of the article (i.e. topsheet), wherein the color difference is measured according to the CIE L*a*b* method as indicated below.

### TEST METHOD: COLOR MEASUREMENT (CIE L*a*b*)

Color measurements on regions of interest are made in accordance with ASTM E1349 using a 45°/0° spectrophotometric color meter suitable for making standard CIE L*a*b* (CIELAB) color measurements. Analyses are performed in a room controlled at about 23 °C ± 2 C° and 50 % ± 2 % relative humidity. Samples are conditioned at the same condition for 2 hours before testing.

A 45°/0° spectrophotometric color meter (X-Rite eXact, X-Rite Inc., Grand Rapids, MI, or equivalent) is used. A measurement port is chosen such that it is small enough to fit within each region of interest to be measured. A measurement port diameter of 2.0 mm may be appropriate. The color meter is configured to output in the CIE L*a*b* color space with the D65 illuminant and a 10° observer.

An article is placed on a flat surface with the top sheet facing upward. The chassis is secured as necessary to a board to hold the article flat. The color meter is used to make 10 individual measurements of L*a*b* color values in the region desired. For each region, average color values are calculated as the arithmetic mean of the ten individual sets of color values.

Differences in color values ΔL*, Δa*, and Δb* between the average color values of the two regions of interest are calculated in accordance with ASTM D2244. ΔL*, Δa*, and Δb* are calculated to the nearest 0.01 and are recorded. Using ΔL*, Δa*, and Δb*, ΔE* is calculated and reported to the nearest 0.01.

### Misc

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular", "optionally" and the likes also qualify features which are not intended to limit the scope of the claims unless specifically indicated to do so.

## Claims

1. An absorbent article (20) for personal hygiene, the article having a wearer-facing side and a garment-facing side, the article comprising:
- a liquid permeable topsheet (24) on the wearer-facing side;
- a liquid impermeable backsheet (25) on the garment-facing side;
- an absorbent layer (60) comprising a superabsorbent polymer and disposed between the topsheet and the backsheet, the absorbent layer being disposed within a core wrap (16, 16');
- a contrasting layer (16) between the topsheet and the backsheet, wherein the contrasting layer is the top layer (16) of the core wrap; and
- a masking layer (54) disposed between the topsheet and the contrasting layer, wherein the masking layer (54) is a fluid handling layer comprising unbonded or loosely bonded fibers and is free of superabsorbent polymer and has a generally white appearance, wherein the masking layer comprises at least one see-through area (86), and the contrasting layer has a color that contrasts with the appearance of the masking layer, so that the contrasting layer is at least partially visible on the wearer-facing side of the article through the see-through area;
wherein the see-through area is a channel area substantially free of masking layer material; and
wherein the see-through area and rest of the masking layer have a ΔE* of at least 2.0 as measured on the wearer-facing side of the article, wherein the color difference is measured according to the CIE L*a*b* space method as indicated herein.

2. An absorbent article according to the preceding claim, wherein the absorbent layer does not comprise channels which are substantially free of absorbent material superposed with the see-through area, in particular wherein the absorbent layer does not comprise channels which are substantially free of absorbent material.

3. An absorbent article according to any of the preceding claims, wherein the unbonded or loosely bonded fibers are cellulosic fibers.

4. An absorbent article according to any of the preceding claims, wherein the topsheet is directly or indirectly bonded to an underlying layer through the see-through areas.

5. An absorbent article according to any of the preceding claims, comprising an acquisition layer (52) between the topsheet and the absorbent layer, wherein the acquisition layer is a nonwoven layer having a basis weight of from about 10 gsm to 60 gsm.

6. An absorbent article according to any of the preceding claims, wherein the absorbent layer comprises superabsorbent polymer particles mixed with cellulose fibers, in particular wherein the absorbent layer comprises from 40% to 80% of superabsorbent particles by weight of the absorbent layer.

7. An absorbent article according to any of the preceding claims, wherein the contrasting layer is a nonwoven layer comprising synthetic fibers, the synthetic fibers comprising a colored pigment.

8. An absorbent article according to any of the preceding claims, wherein the contrasting layer comprises an ink printed at least in the areas corresponding to the see-through areas.

9. An absorbent article according to any of the preceding claims, wherein the see-through area and rest of the masking layer have a maximum ΔE* of at least 2.0 as measured on the wearer-facing side of the article, wherein the color difference is measured according to the CIE L*a*b* space method as indicated herein.

10. A package comprising a plurality of absorbent articles according to any of the preceding claims.

## Patentansprüche

1. Absorptionsartikel (20) für Körperpflege, wobei der Artikel eine trägerseitige Seite und eine zum Kleidungsstück zeigende Seite aufweist, der Artikel umfassend:
- eine flüssigkeitsdurchlässige Oberschicht (24) auf der trägerseitigen Seite;
- eine flüssigkeitsundurchlässige Unterschicht (25) auf der zum Kleidungsstück zeigenden Seite;
- eine Absorptionsschicht (60), umfassend ein Superabsorber-Polymer und angeordnet zwischen der Oberschicht und der Unterschicht, wobei die Absorptionsschicht innerhalb einer Kernumwicklung (16, 16') angeordnet ist;
- eine Kontrastschicht (16) zwischen der Oberschicht und der Unterschicht, wobei die Kontrastschicht die Oberschicht (16) der Kernumwicklung ist; und
- eine Maskierungsschicht (54), die zwischen der Oberschicht und der Kontrastschicht angeordnet ist, wobei die Maskierungsschicht (54) eine Flüssigkeitshandhabungsschicht ist, umfassend ungebundene oder lose gebundene Fasern und die frei von Superabsorber-Polymer ist und ein allgemein weißes Erscheinungsbild aufweist, wobei die Maskierungsschicht wenigstens einen durchsichtigen Bereich (86) umfasst und die Kontrastschicht eine Farbe aufweist, die mit dem Erscheinungsbild der Maskierungsschicht in Kontrast steht, sodass die Kontrastschicht auf der trägerseitigen Seite des Artikels durch den durchsichtigen Bereich wenigstens teilweise sichtbar ist;
wobei der durchsichtige Bereich ein Kanalbereich ist, der im Wesentlichen frei von Maskierungsschichtmaterial ist; und
wobei der durchsichtige Bereich und der Rest der Maskierungsschicht ein ΔE* von wenigstens 2,0 aufweisen, gemessen auf der trägerseitigen Seite des Artikels, wobei der Farbunterschied gemäß der hierin angegebenen CIE L*a*b*-Abstandsmethode gemessen wird.

2. Absorptionsartikel nach dem vorstehenden Anspruch, wobei die Absorptionsschicht keine Kanäle umfasst, die im Wesentlichen frei von Absorptionsmaterial sind und die über dem durchsichtigen Bereich angeordnet sind, insbesondere wobei die Absorptionsschicht keine Kanäle umfasst, die im Wesentlichen frei von Absorptionsmaterial sind.

3. Absorptionsartikel nach einem der vorstehenden Ansprüche, wobei die ungebundenen oder lose gebundenen Fasern Cellulosefasern sind.

4. Absorptionsartikel nach einem der vorstehenden Ansprüche, wobei die Oberschicht direkt oder indirekt an eine Unterlegschicht durch die durchsichtigen Bereiche gebunden ist.

5. Absorptionsartikel nach einem der vorstehenden Ansprüche, umfassend eine Aufnahmeschicht (52) zwischen der Oberschicht und der Absorptionsschicht, wobei die Aufnahmeschicht eine Vliesschicht ist, die ein Basisgewicht von etwa 10 Gramm pro Quadratmeter bis 60 Gramm pro Quadratmeter aufweist.

6. Absorptionsartikel nach einem der vorstehenden Ansprüche, wobei die Absorptionsschicht Superabsorberpolymerteilchen umfasst, die mit Cellulosefasern vermischt sind, insbesondere wobei die Absorptionsschicht von 40 Gew.-% bis 80 Gew.-% superabsorbierende Teilchen umfasst, bezogen auf die Absorptionsschicht.

7. Absorptionsartikel nach einem der vorstehenden Ansprüche, wobei die Kontrastschicht eine Vliesschicht ist, umfassend synthetische Fasern, die synthetischen Fasern umfassend ein Farbpigment.

8. Absorptionsartikel nach einem der vorstehenden Ansprüche, wobei die Kontrastschicht eine Tinte umfasst, die wenigstens in den Bereichen aufgedruckt ist, die den durchsichtigen Bereichen entsprechen.

9. Absorptionsartikel nach einem der vorstehenden Ansprüche, wobei der durchsichtige Bereich und der Rest der Maskierungsschicht ein maximales ΔE* von wenigstens 2,0 aufweisen, gemessen auf der trägerseitigen Seite des Artikels, wobei der Farbunterschied gemäß der hier angegebenen CIE L*a*b*-Abstandsmethode gemessen wird.

10. Verpackung, umfassend eine Vielzahl von Absorptionsartikeln nach einem der vorstehenden Ansprüche.

## Revendications

1. Article absorbant (20) destiné à une hygiène personnelle, l'article ayant un côté tourné vers l'utilisateur et un côté tourné vers le vêtement, l'article comprenant :
- une feuille supérieure perméable aux liquides (24) sur le côté tourné vers l'utilisateur ;
- une feuille arrière imperméable aux liquides (25) sur le côté tourné vers le vêtement ;
- une couche absorbante (60) comprenant un polymère superabsorbant et disposée entre la feuille supérieure et la feuille arrière, la couche absorbante étant disposée à l'intérieur d'une enveloppe centrale (16, 16') ;
- une couche de contraste (16) entre la feuille supérieure et la feuille arrière, la couche de contraste étant la couche supérieure (16) de l'enveloppe centrale ; et
- une couche de masquage (54) disposée entre la feuille supérieure et la couche de contraste, dans lequel la couche de masquage (54) est une couche de manipulation de fluides comprenant des fibres non liées ou faiblement liées, et est exempte de polymère superabsorbant et d'apparence généralement blanche, dans lequel la couche de masquage comprend au moins une zone transparente (86), et la couche de contraste a une couleur qui contraste avec l'apparence de la couche de masquage, de sorte que la couche de contraste est au moins partiellement visible sur le côté de l'article faisant face à l'utilisateur à travers la zone transparente ;
dans lequel la zone transparente est une zone de canal pratiquement exempte de matériau de la couche de masquage ; et
dans lequel la zone transparente et le reste de la couche de masquage ont un ΔE* d'au moins 2,0 mesuré sur la face de l'article faisant face à l'utilisateur, dans lequel la différence de couleur est mesurée selon le procédé de l'espace L*a*b* de la CIE, comme indiqué dans le présent document.

2. Article absorbant selon la revendication précédente, dans lequel la couche absorbante ne comprend pas de canaux sensiblement exempts de matériau absorbant superposés à la zone transparente, en particulier, dans lequel la couche absorbante ne comprend pas de canaux sensiblement exempts de matériau absorbant.

3. Article absorbant selon l'une quelconque des revendications précédentes, dans lequel les fibres non liées ou faiblement liées sont des fibres cellulosiques.

4. Article absorbant selon l'une quelconque des revendications précédentes, dans lequel la feuille supérieure est directement ou indirectement liée à une couche sous-jacente à travers les zones transparentes.

5. Article absorbant selon l'une quelconque des revendications précédentes, comprenant une couche d'acquisition (52) entre la feuille supérieure et la couche absorbante, dans lequel la couche d'acquisition est une couche non tissée ayant un poids de base d'environ 10 g/m² à 60 g/m².

6. Article absorbant selon l'une quelconque des revendications précédentes, dans lequel la couche absorbante comprend des particules de polymère superabsorbant mélangées à des fibres de cellulose, en particulier dans lequel la couche absorbante comprend de 40 % à 80 % de particules superabsorbantes en poids de la couche absorbante.

7. Article absorbant selon l'une quelconque des revendications précédentes, dans lequel la couche contrastante est une couche non tissée comprenant des fibres synthétiques, les fibres synthétiques comprenant un pigment coloré.

8. Article absorbant selon l'une quelconque des revendications précédentes, dans lequel la couche contrastante comprend une encre imprimée au moins dans les zones correspondant aux zones transparentes.

9. Article absorbant selon l'une quelconque des revendications précédentes, dans lequel la zone transparente et le reste de la couche de masquage ont un ΔE* maximal d'au moins 2,0, mesuré sur la face de l'article faisant face à l'utilisateur, dans lequel la différence de couleur est mesurée selon le procédé de l'espace L*a*b* de la CIE, comme indiqué dans le présent document.

10. Conditionnement comprenant une pluralité d'articles absorbants selon l'une quelconque des revendications précédentes.
